# EUROPÄISCHE PATENTANMELDUNG

(11) **EP 0 807 688 A2**
(43) Veröffentlichungstag der Anmeldung: **19.11.1997**
(21) Anmeldenummer: 97107742.5
(22) Anmeldetag: 12.05.1997
(51) Int. Cl.: C12N 15/85, A01K 67/027, C12N 15/88, C12N 5/10, A61K 31/70, A61K 48/00

(54) **Nukleinsaürekonstrukt zur spezifischen Genexpression in glatten Gefässmuskelzellen**

(30) Priorität: 10.05.1996 DE 9620308 U
(71) Anmelder: Franz, Wolfgang-M., Dr., 23627 Gross Grönau (DE)
(72) Erfinder: Franz, Wolfgang-M.Dr., D-23627 Gross Grönau (DE); Müller, Olivier, D-69115 Heidelberg (DE); Katus H.A. Prof. Dr., D-23909 Ratzeburg (DE)
(74) Vertreter: Kinzebach, Werner, Dr.

(57) **Zusammenfassung**

Die Erfindung betrifft neuartige Nukleinsäurekonstrukte umfassend einen eine regulative SMHC-Sequenz, welche insbesondere in vivo eine spezifische Genexpression in der glatten Gefäßmuskulatur adulter Säuger ermöglichen; Vektoren, welche diese Konstrukte umfassen; die Verwendung dieser Nukleinsäurekonstrukte zur gentherapeutischen Behandlung von Dysfunktionen der glatten Gefäßmuskulatur; Mittel enthaltend die erfindungsgemäßen Konstrukte und Vektoren; sowie transgene Tiere und Zellinien, welche diese Nukleinsäurekonstrukte oder Vektoren tragen.

## Beschreibung

Die Erfindung betrifft neuartige Nukleinsäurekonstrukte, welche insbesondere in vivo eine spezifische Genexpression in der glatten Gefäßmuskulatur adulter Säuger ermöglichen; Vektoren, welche diese Konstrukte umfassen; die Verwendung dieser Nukleinsäurekonstrukte zur gentherapeutischen Behandlung von Dysfunktionen der glatten Gefäßmuskulatur; Mittel enthaltend die erfindungsgemäßen Konstrukte und Vektoren; sowie transgene Tiere und Zellinien, welche diese Nukleinsäurekonstrukte oder Vektoren tragen. Anwendungsgebiete der Erfindung sind die Medizin, die Gentechnik und die pharmazeutische Industrie.

Die glatte Gefäßmuskulatur bildet den Hauptbestandteil der Gefäßwände arterieller und venöser Blutgefäße. Sie ist an der Pathogenese vieler das Gefäßsystem betreffender Erkrankungen beteiligt, die trotz intensiver Forschung bis zum heutigen Tag nicht oder nur unzureichend therapierbar sind. Bei der Entstehung von vaskulären Läsionen im Rahmen der Atherosklerose, Restenose nach PTCA (Perkutane Transluminale Coronar Angioplastie) und arteriellen Hypertonie spielt das pathologische Wachstum von glatten Muskelzellen der Gefäßwand eine zentrale Rolle (Lüscher, T.F. (1995), Schweiz Med Wochenschrift 125: 270-282). Darüber hinaus sind eine zunehmende Zahl von genetisch bedingten, d.h. angeborenen, Erkrankungen bekannt, welche eine Schädigung der Gefäßwand verursachen. Hierzu zählen beispielsweise die supravalvuläre Aortenstenose und das Williams Syndrom (Keating, M.T. und Sanguinetti, M.C. (1996), Science 272:681-5).

Im Rahmen der Hypertonie kann eine Tonussteigerung der glatten Gefäßmuskulatur beobachtet werden. Im weiteren Verlauf werden im Bereich der Widerstandsgefäße auch zur Mediaverdickung führende Umbauvorgänge (Remodeling) beobachtet, während in den großen Überleitungsgefäßen eher atherosklerotische Veränderungen gefunden werden. Bei der Atherosklerose tritt, wie auch bei der Restenose nach PTCA und Alterungsprozessen, eine Proliferation und Migration glatter Muskelzellen auf. Diese Zellen zeigen eine Dedifferenzierung vom kontraktilen zum sekretorischen Phänotyp. Dadurch ergeben sich auch Veränderungen in der Expression von in der glatten Muskulatur vorkommenden Genen. Die Pathogenese dieser Störungen ist noch nicht geklärt; hierzu würde sich ein transgenes Tiermodell anbieten, das die selektive Expression von Kandidatengenen in der glatten Gefäßmuskulatur erlaubt.

Während es für die arterielle Hypertonie pharmakologische Therapiemöglichkeiten gibt, gelten andere Dysfunktionen der glatten Gefäßmuskulatur, wie die Restenose nach PTCA, als schwer therapiebar. Aus diesem Grund wurden verschiedene therapeutische DNA-Sequenzen getestet, die alle einen antiproliferativen Effekt auf die glatte Gefäßmuskulatur gemeinsam haben (Gibbons, G.H. und Dzau, V.J. (1996), Science 272:689-93). Einer Anwendung dieser DNA-Sequenzen im Rahmen einer Therapiestunde am Menschen stehen jedoch Bedenken hinsichtlich der Sicherheit der Genexpression im gewünschten Zielgewebe, der glatten Gefäßmuskulatur, entgegen. Es ist nämlich bisher noch nicht gelungen, eine regulatorische DNA-Sequenz bereitzustellen, die eine spezifische Expression in somatischen Zellen der glatten Gefäßmuskulatur von Arterien und Venen adulter Säuger ermöglicht. Es besteht also das Risiko einer Koexpression von therapeutischen Genen in anderen Zellen als der glatten Gefäßmuskulatur, was zu unvorhersehbaren Folgen bei einer somatischen Gentherapie führen könnte.

Es gibt wenige Arbeiten, die sich mit der in vivo Charakterisierung von regulatorischen DNA-Sequenzen beschäftigen. Während einige Promotoren mit Herz-spezifischer Aktivität gefunden und deren Gewebespezifität im transgenen Tiermodell nachgewiesen wurde (Franz, W.M. et al. (1995), Z. Kardiolo 84: Suppl, 4, 17-32; PCT/DE 96/02181), sind nur in wenigen Fällen regulatorische Sequenzen auf ihre in vivo Spezifität für das Gefäßsystem adulter Säuger untersucht worden. Der Präproendothelin-Promotor ermöglicht beispielsweise eine hohe Expression in der Gefäßwand transgener Mäuse, erlaubt aber keine Differenzierung zwischen Endothel und glatter Muskulatur (Harats, D. et al. (1995). J.Clin.Inv. 95: 1335-1344). Daher würde dieser Promotor beim kathetervermittelten Gentransfer keinen Vorteil gegenüber der Verwendung starker unspezifischer Promotoren, wie z.B. dem CMV-Promotor, bieten.

Weitere Promotoren mit Aktivität in der glatten Gefäßmuskulatur sind der Smooth Muscle α-Aktin- und der Calponin-h1-Promotor, die jedoch nur in der Zellkultur getestet wurden. (Shimizu, R.T. et al. (1995), J. Biol. Chem. 270: 7631-7643; Miano, J.M. und Olson, E.N. (1996), J. Biol. Chem. 271: 7095-7103). In vitro-Untersuchungen der Gewebespezifität von Promotoren erlauben jedoch keinerlei zuverlässigen Rückschlüsse auf die in vivo-Eigenschaften der Promotoren im adulten Lebewesen.

Ein Beispiel für die limitierte Zuverlässigkeit von Zellkulturexperimenten bei der Analyse von Promotoren ist die beobachtete Diskrepanz der Untersuchungsergebnisse zur Gewebespezifität des a1(I) Kollagen Promotors in Zellkultur bzw. im transgenen Tiermodell (Bedalov, A. et al. (1994), J. Biol. Chem. 269: 4903-9). Während bei der Transfektion von primären Gefäßmuskelzellen mit einem an den Promotorabschnitt gekoppelten CAT-Reportergen hohe CAT-Aktivitäten in vitro beobachtet wurden, konnten in der Aorta transgener Tiere nur eine viel geringere Expression des Reportergens nachgewiesen werden als in den Sehnen oder Knochen des Tiers, obwohl das endogene Gen in allen drei Geweben stark exprimiert wird. Ein weiterer Nachteil von Promotoranalysen in Zellkulturexperimenten liegt in der fehlenden Überprüfbarkeit auf unspezifische Expression des Reportergens, bedingt durch eine unzureichende Promotorsequenz im Falle negativ regulatorischer Elemente. So zeigte sich bei der Untersuchung eines Promotorabschnittes des von Willebrand-Faktors in zwei voneinander unabhängigen Linien eine überraschende Reportergenexpression im Gehirn. Obwohl dieses Konstrukt in kultivierten Endothelzellen aktiv war, blieb die in vivo Expression neben der unspezifischen Gehirnexpression auf das Gefäßendothel im Gehirnbereich beschränkt (Aird et al. (1995), Proc. Natl. Acad. Sci. U.S.A. 92:4567-4571).

Zum gewebespezifischen Gentransfer ist eine regulatorische DNA-Sequenz erforderlich, die zwei Anforderungen in vivo erfüllen muß: Einerseits muß eine ausreichend hohe Expressionsstärke erreicht werden, andererseits ist eine möglichst hohe Gewebespezifität erforderlich. Es ist bisher noch nicht gelungen, eine regulatorische DNA-Sequenz bereitzustellen, welche diesen Anforderungen in vivo genügt und für die spezifische Expression in somatischen Zellen der glatten Gefäßmuskulatur von Arterien und Venen verwendbar ist.

Es gibt zwar schon eine Reihe von Therapieansätzen für Restenose durch liposomalen oder viralen DNA-Transfer (Schott, E. und Paul, M. (1996), Internist 37: 350-359). Eine Anwendung in Therapiestunden am Menschen ist jedoch aufgrund von Sicherheitserwägungen nicht möglich, da die bisher verwendeten regulatorischen Promotorsequenzen auch in anderen Geweben stark aktiv sind. Aus diesem Grund besteht bei den bisherigen Therapieansätzen das Risiko einer Koexpression von therapeutischen Genen in anderen Zellen als der glatten Gefäßmuskulatur.

Kürzlich wurde ein transgenes Tiermodell beschrieben, das die 5'-regulatorische Sequenz des SM22α-Gens charakterisiert. Es wurde eine Expression beobachtet, welche auf die glatte Gefäßmuskulatur der Arterienwände beschränkt war (vgl. Miano, J.M. und Olson, E.N. (1996) J. Biol.Chem.; 271:7095-7103). Es wurde jedoch keine Expression in Venen oder Koronararterien nachgewiesen. Dieses Tiermodell besitzt somit keine zufriedenstellenden Eigenschaften für ein Modellsystem zur Untersuchung von Dysfunktionen der glatten Gefäßmuskulatur. Darüber hinaus stellt es keine erfolgversprechende Ausgangsbasis für ein gentherapeutisches Behandlungssystem von Dysfunktionen der glatten Gefäßmuskulatur dar.

Es besteht somit ein dringender Bedarf an verbesserten Nukleinsäurekonstrukten, die eine spezifische gentherapeutische Behandlung von Dysfuktionen der glatten Gefäßmuskulatur im adulten Säuger, insbesondere dem Menschen, ermöglichen. Darüber hinaus wäre es zur Entwicklung neuer Therapieformen für solche Dysfuktionen von Vorteil, ein geeignetes transgenes Tiermodell zu entwickeln, mit dessen Hilfe die Pathogenese solcher Dysfunktionen entschlüsselt und genetische Einflüsse auf die Entstehung der Erkrankung untersucht werden können. Außerdem wären spezifisch transformierte Zellinien zur Etablierung von in vitro Testsystemen für die Suche neuer therapeutisch wirksamer Substanzen zur Behandlung solcher Dysfunktionen nützlich.

Der Erfindung lag deshalb die Aufgabe zugrunde, ein Nukleinsäurekonstrukt für den gezielten in vivo Gentransfer zu oder die gezielte, möglichst spezifische in vivo Genexpression in glatten Gefäßmuskelzellen adulter Säuger bereitzustellen. Insbesondere sollte Transfer oder Expression nicht auf einzelne Teilbereiche des Gefäß-Systems, wie Arterien oder Venen, beschränkt sein. Eine weitere Aufgabe der Erfindung bestand in der Bereitstellung von transgenen Tiermodellen und Zellinien zur Etablierung von in vivo bzw. in vitro Testsystemen für Untersuchungen im Zusammenhang mit Dysfunktionen glatter Gefäßmuskelzellen, insbesondere im Zusammenhang mit Restenose. Außerdem lag der Erfindung die Aufgabe zugrunde, ein gentherapeutisches Konzept zur Behandlung von Dysfunktionen der glatten Gefäßmuskeln und insbesondere zur Hemmung der Prolifertion glatter somatischer Gefäßmuskelzellen aufzubauen, welches in vivo maximale Sicherheit vor unkontrollierter Expression in anderen Zellen als den glatten Gefäßmuskelzellen bietet.

Überraschenderweise wurden obige Aufgaben gelöst durch Bereitstellung eines Nukleinsäurekonstrukts mit Spezifität für glatte Gefäßmuskelzellen adulter Säuger, das dadurch gekennzeichnet ist, daß es unter der Kontrolle einer 5'-regulativen Nukleotidsequenz für die Myosin-Schwere Kette der glatten Muskulatur (SMHC) eine funktionale Nukleotidsequenz umfaßt; sowie durch Bereitstellung von Vektorsystemen, welche ein solches Konstrukt, insbesondere eine Expressionskassette, umfassen, und transgene Tiere sowie Zellinien, welche durch Einschleusung eines solchen Nukleinsäurekonstruktes erhältlich sind.

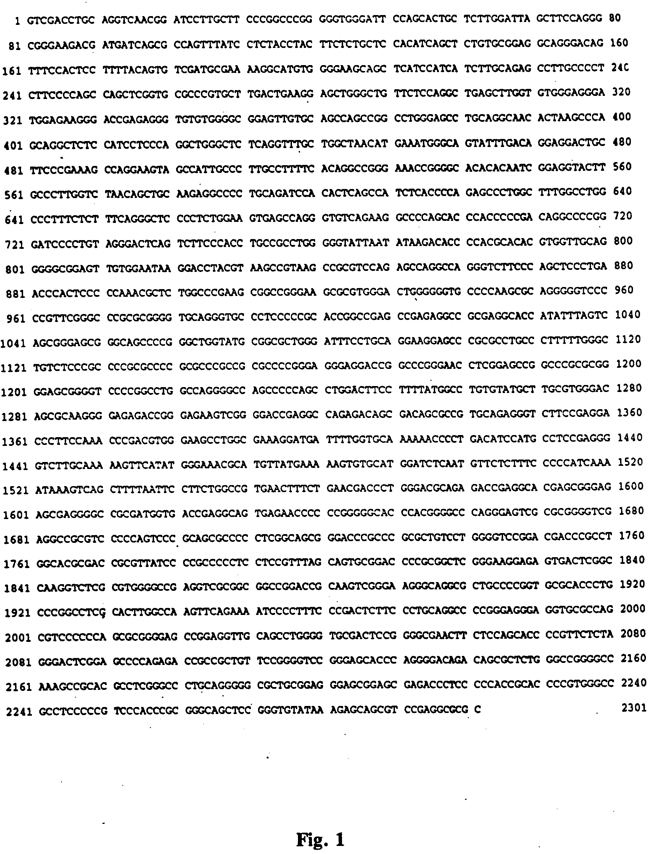

Überraschenderweise wurde festgestellt, daß der Gentransfer unter Kontrolle eines 5'-SMHC Promotors insbesondere in vivo im adulten Säuger gefäßspezifisch erfolgt. So wurde erstmals gezeigt, daß die Luciferase-Expression unter Kontolle der 2.301 kb langen SMHC Promotorsequenz (Figur 1, -2305bp bis -4bp stromaufwärts vom Transkriptionsstart des SMHC-Gens) aus Kaninchen in transgenen Mäusen gewebespezifisch ist.

Somit wird überraschenderweise mit Hilfe der erfindungsgemäßen Nukleinsäurekonstrukte eine spezifische Genexpression in vaskulärem glatten Muskelgewebe ermöglicht, während in viszeralem glattem Muskelgewebe nur Hintergrund-Aktivitäten nachweisbar ist. Im Gegensatz zum Stand der Technik wird somit ein in vivo Gentransfer in adulten Säugern auf arterielle und venöse glatte Gefäßmuskulatur einschließlich der glatten Gefäßmuskulatur der Koronararterien, ermöglicht.

Die erfindungsgemäßen Nukleinsäurekonstrukt eignen sich somit zum gewebespezifischen Transfer einer funktionalen Nukleotidsequenz, die
a) für wenigstens ein in den Gefäßmuskelzellen zu exprimierendes heterologes oder homologes Genprodukt kodiert; oder
b) eine therapeutisch wirksame Nukleotidsequenz umfaßt.

Unter einer therapeutischen oder gentherapeutischen Nukleinsäuresequenz im Sinne der Erfindung versteht man ein Nukleinsäurekonstrukt mit einer Nukleinsäuresequenz, die insbesondere eine DNA- oder RNA-Sequenz, vorzugsweise eine einzelsträngige oder doppelsträngige, vor allem eine doppelsträngige DNA-Sequenz ist, wobei das Nukleinsäurekonstrukt als Arzneimittl für die gentherapeutische Behandlung von Gefäßerkrankungen, insbesondere zur Behandlung von Restenose der Blutgefäße in vorteilhafter Weise verwendet werden kann.

Vorzugsweise wird als therapeutisches Gen eine Nukleotidsequenz verwendet, die beispielsweise in ihrer Wirkung die Proliferation der glatten Gefäßmuskulatur hemmt. Dabei kann es sich beispielsweise um eine Antisense-Nukleinsäure, wie z.B. ein Antisense-Oligonukleotid, vorzugsweise Antisense-DNA-Nukleotid oder ein Ribozym handeln, welche die Expression von Genen im erkrankten Gefäß erhöht, verringert oder unterbindet, um so die Erkrankung zu therapieren (vgl. z.B. Barr,E. und Leiden, J.M. (1994) Trends Cardiovasc. Med. 4, 57-63; Bertrand, E. et al (1994) Nucleic Acids Res., 22, 293-300).

Das erfindungsgemäße Nukleinsäurekonstrukt wird vorzugsweise mit einem Virusvektor und/oder mit Liposomen kombiniert, wie z.B mit einem Adenovirusvektor.

Eine "gewebespezifische" oder "gefäßspezifische" Expression im Sinne der vorliegenden Erfindung bedeutet, daß die zu transferierende therapeutische Nukleotidsequenz oder das exprimierende Genprodukt insbesondere im Bereich der glatten Gefäßmuskulatur von Venen und Arterien adulter Säuger nachgewiesen werden kann. "Spezifität" im Sinne der vorliegenden Erfindung bedeutet nicht die ausschließliche Nachweisbarkeit in einem einzelnen Gewebetyp oder Organ. Vielmehr gilt das erfindungsgemäße Spezifitätskriterium als erfüllt, wenn das zu exprimierende Genprodukt oder die therapeutische Nukleotidsequenz im Zielgewebe, d.h. den glatten, vorzugsweise somatischen, Gefäßmuskelzellen deutlich erhöhte Konzentrations- oder Aktivitätswerte gegenüber Nicht-Zielgewebe aufweist.

Die Spezifität erfindungsgemäßer Konstrukte kann durch Expression von Markergenen, wie z.B. dem Enzym Luciferase, leicht überprüft werden. Der Fachmann erkennt anhand der vorliegenden Versuchsergebnisse, daß ein Vergleich der für einzelne "Referenz"-Gewebe ermittelten Luciferase-Aktivitäten eine Aussage über die Spezifität eines Konstruktes ermöglicht. Das erfindungsgemäße Spezifitätskriterium ist beispielsweise erfüllt, wenn die Luciferase-Aktivität, gemessen in Lichteinheiten (LU) bezogen auf Milligramm Gesamtprotein der untersuchten Gefäßmuskelprobe aus wenigstens einem positiven venösen und arteriellen "Referenz"-Gewebe signifikant höher ist als Luciferase-Aktivität in wenigstens einem negativen "Referenz"-Gewebe. Ein positives Referenzgewebe ist beispielsweie ausgewählt unter Aorta abdominalis, Aorta thoracica, Arteria renalis, Vena cava inferior, Vena portae, Arteria pulmonalis, Arteria femoralis, Arteria iliaca, Arteria coronaria cordis, und Mesenterium, insbesondere Aorta thoracica, Aorta abdominalis und Vena cava inferior. Ein negatives Referenzgewebe ist beispielsweise ausgewählt unter Myokard, Leber, Milz, Diaphragma, Uterus, Skelettmuskel und Ösophagus, insbesondere Skelettmuskel, Ösophagus und Myokard.

Eine signifikante Erhöhung liegt vor, wenn die Luciferase-Aktivität (in LU/mg Protein) im positiven Referenzgewebe einen mindestens dreifach erhöhten Wert besitzt. Die gewöhnlich zu beobachtenden Luciferase-Werte sind aber um mehr als das zehn- bis eintausend-fache, wie z.B. um das zehn- bis einhundert-fache oder mehr, im positiven Referenzgewebe erhöht. Umgerechnet in exprimiertes Luciferase-Protein sind (z.B. für Aorta-Gewebe, dessen Gesamtproteingehalt zu etwa einem Drittel bis etwa einem Viertel oder einem Fünftel aus glatten Muskelzellen stammt) Konzentrationen im Bereich von etwa 2 bis 60 pg, wie z.B. etwa 5 bis 15 pg oder insbesondere etwa 8 bis 10 pg, Luciferase pro Milligramm Gesamtprotein (bei einem Background von etwa 0 pg Luciferase/mg Gesamtprotein) errechenbar. Entspechende Werte (in pg/mg bzw. umgerechnet z.B. in nmol/mg) sind bei Expression anderer Genprodukte mit dem erfindungsgemäßen Expressionssystem zu erwarten. Damit entspricht der mit dem erfindungsgemäßen System erzielbare Expressionsgrad den werten, die man bei Expression mit normalen "Household"-Promotoren in etwa beobachtet bzw. erwartet (nämlich etwa 40 bis 50pg exprimiertes Protein pro Milligramm Gesamtprotein).

Die erfindungsgemäß bevorzugten Vektorsysteme, wie z.B. Expressionssysteme, zum spezifischen in vivo Gentransfer und/oder - expression in glatten Gefäßmuskelzellen adulter Säuger sind z.B. Mikroinjektions- oder Transfektionskonstrukte oder virale oder nichtvirale Genshuttles, worin ein beliebiges homologes oder heterologes Gen bzw. ein beliebiges funktionelles homologes oder heterologes Nukleinsäuregfragment gekoppelt an die regulatorische DNA-Sequenz von SMHC, einkloniert ist.

Die erfindungsgemäß verwendete kodierende Nukleotidsequenz kodiert insbesondere für ein Markergen, wie z.B. Luciferase oder β-Galactosidase, oder ein therapeutisch wirksames, vorzugsweise antiproliferativ wirkendes, Genprodukt. Das antiproliferativ wirkende Genprodukt ist beispielsweise ausgewählt unter Toxinen und Zellzyklus-Inhibitoren.

Erfindungsgemäß bevorzugte Nukleinsäurekonstrukte umfassen in ihrer regulativen Nukleotidsequenz einen SMHC-Promotor. Ein besonders bevorzugter SMHC-Promotor besitzt die in Figur 1 angegebene Mukleotidsequenz von Nukleotidrest +1 bis Nukleotidrest +2301 (-2305 bp bis -4 bp), gerechnet vom Transkriptionsstart) oder stellt ein funktionales Äquivalent davon dar.

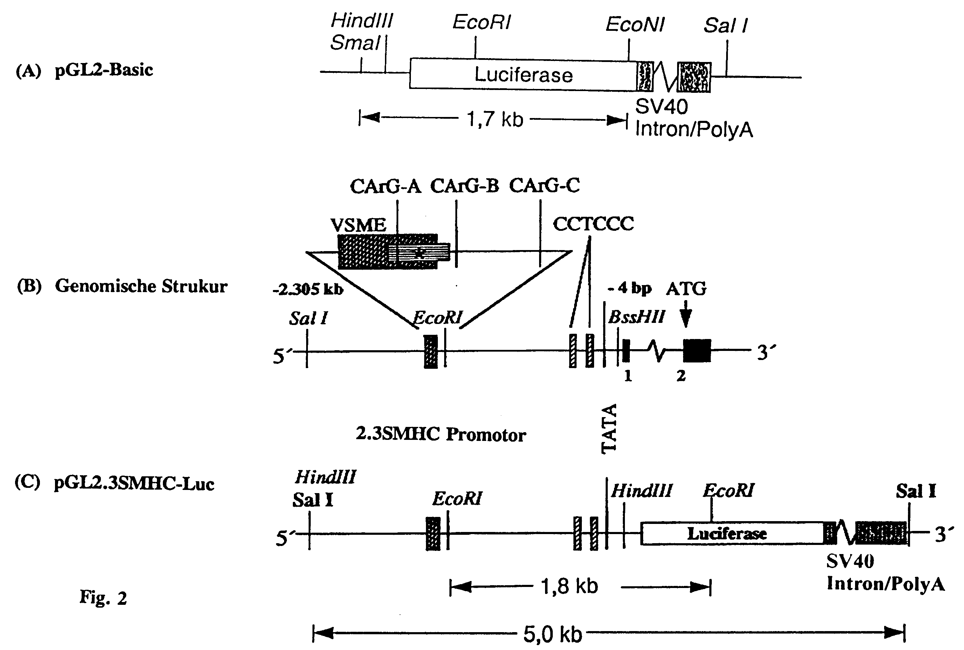

Wesentliche regulative Elemente der erfindungsgemäßen bevorzugt verwendeten Promotorsequenz liegen, ohne darauf beschränkt zu sein, in der Region von -24 bp bzw. -2140 bp. So ist z.B. eine TATA-Box bei - 24 bp zu finden, CCTCCC-Sequenzen, die für die Transkriptionsaktivität in glatten Muskelzellen wichtig sind, liegen bei -63 bp und -89 bp. Weitere wichtige Teilsequenzen liegen bei etwa -1235 bis -1332 bp. In diesem Bereich liegt eine spezifische Enhancersequenz (VSME; vgl. Fig.2) und es werden dort weitere regulative Sequenzen, wie ein negativ regulatives Element, vermutet, wovon einzelne für die beobachtete Nicht-Expression von Luciferase in nicht-glatten Muskelzellen verantwortlich sein könnten.

Die erfindungsgemäßen Nukleinsäurekonstrukte können weitere übliche regulative Nukleotidsequenzen umfassen, wie z.B. Leadersequenzen, Enhancersequenzen, Polyadenylierungssignale und die Expressionsrate mengenmäßig oder in ihrem zeitlichen Verlauf steuernde Sequenzen. Ein bevorzugtes Konstrukt umfaßt in 3'-Position zur funktionalen Sequenz ein SV40-Polyadenylierungssignal.

Ein funktionales Äquivalent einer regulativen Nukleotidsequenz, wie z.B. des in Figur 1 gezeigten SMHC-Promotors aus Kaninchen, kann beispielsweise aus einem beliebigen anderen Säuger abgeleitet sein, wie z.B. von Maus, Ratte, Hamster, Katze, Hund, Schwein, Schaf, Ziege, Rind, Pferd, Affe oder Mensch. Voraussetzung ist jedoch, daß eine vergleichbare vorteilhafte Gewebespezifität der Genexpression (z.B. nachgewiesen mit Luciferase als Markergen) in vivo im adulten Säuger erreichbar ist. Gleiches gilt für Varianten einer natürlichen SMHC-Promotorsequenz, die man durch ein- oder mehrfache Nukleotidaddition, -insertion, -substitution oder -deletion erhält. Wie die erfindungsgemäßen Versuchsergebnisse belegen, besitzen die erfindungsgemäß verwendeten regulativen Nukleotidsequenzen alle regulatorischen Elemente, die für einen selektiven Gentransfer auf bzw. eine selektive Genexpression in glatten Gefäßmuskelzellen in vivo in adulten Säugern erforderlich sind. Auf der Basis der erfindungsgemäßen Versuchsergebnisse kann der Fachmann unter Befolgung der erfindungsgemäßen Lehre weitere funktional äquivalente regulative Nukleotidsequenzen bereitstellen, welche die hierin beschriebenen Spezifitätskriterien erfüllen.

Unter einem funktionalen Äquivalent einer erfindungsgemäß verwendeten therapeutischen Nukleotidsequenz versteht man eine durch ein- oder mehrfache Nukleotidaddition, -insertion, -substitution oder -deletion erhaltene oder natürlich vorkommende Variante einer konkreten Sequenz, welche im wesentlichen die gleiche Funktion wie die nicht-modifizierte Nukleotidsequenz aufweist.

Obige Ausführungen zur funktionalen Äquivalenz gelten entsprechend für funktionale Äquivalente von erfindungsgemäßen brauchbaren, kodierenden Nukleotidsequenzen, wobei die Änderungen in der Nukleotidsequenz gegebenenfalls Additionen, Insertionen, Substitutionen oder Deletionen einzelner oder mehrerer Aminosäuren im resultierenden Genprodukt bewirken. Die Funktion des Genproduktes im Organismus, wie z.B. der antiproliferative Effekt auf Gefäßmuskelzellen, bleibt jedoch nach der Modifikation im wesentlichen erhalten, kann aber auch erhöht oder in gewissem Umfang verringert werden.

Als Bestandteil eines Mikroinjektions- bzw. Transfektionskonstrukts werden erfindungsgemäße Vektorsysteme zur Erstellung eines transgenen Tiermodells (durch Genaddition oder durch Gendeletion) eingesetzt. Als Bestandteil eines Transfektionskonstrukts sind sie beispielsweise zur Modifikation embryonaler Stammzellen brauchbar.

Außerdem dienen die erfindungsgemäßen Konstrukte zur Erstellung und Charakterisierung transgener Tierlinien und zur ex vivo Untersuchung der Spezifität, Stabilität und Effizienz der Genexpression von Markergenen, wie Luciferase und β-Galaktosidase, und therapeutisch wirksamen Genprodukten.

Auch können die selektierten und/oder immortalisierten glatten Gefäßmuskelzellen zur zellvermittelten Gentransplantation und zum somatischen Gentransfer eingesetzt werden.

Ein weiter Gegenstand der Erfindung sind somit transgene Säugetiere, die transformiert sind mit wenigstens einem erfindungsgemäßen Nukleinsäurekonstrukt. Transgene Säugetiere sind z.B. ausgewählt unter Maus, Ratte, Hamster, Kaninchen, Katze, Hund, Schwein, Schaf, Ziege, Rind, Pferd, Affe, insbesondere Maus und Ratte.

Gegenstand der Erfindung ist auch ein Verfahren zur Herstellung solcher transgener Säugetiere, wobei man eine befruchtete Eizelle des Säugetiers gewinnt; in den männlichen Pronucleus eine Lösung mikroinjiziert, welche wenigstens ein erfindungsgemäßes Nukleinsäurekonstrukt oder wenigstens einen erfindungsgemäßen Vektor umfaßt; die transfizierte Eizelle in das Ovidukt eines scheinträchtigen Wirts implantiert und das transgene Tier durch den Wirt austragen läßt.

Weiterhin werden erfindungsgemäße Konstrukte zur Selektion und/oder Immortalisierung von glatten Gefäßmuskelzellen aus embryonalen Stammzellen eingesetzt und dienen somit der Etablierung eines in vitro Modells. Ein solches in vitro Modell wird erfindungsgemäß zur Untersuchung von potentiellen therapeutisch wirksamen Substanzen, insbesondere für pharmakotoxische Untersuchungen angewendet.

Die selektierten und/oder immortalisierten glatten Gefäßmuskelzellen können zuammen mit einem oder mehreren therapeutisch wirksamen Genen durch ein virales oder nichtvirales Genshuttle vor einer Transplantation in einen Organismus ex vivo transfiziert werden.

Ein weiterer Gegenstand der Erfindung sind somit, vorzugsweise somatische, Zellinien der glatten Gefäßmuskulatur, die transformiert sind mit einer Nukleotidsequenz, umfassend wenigstens ein erfindungsgemäßes Nukleinsäurekonstrukt. Solche Zellinien sind abgeleitet aus embryonalen Stammzellen von Maus, Ratte, Hamster, Kaninchen, Katze, Hund, Schwein, Schaf, Ziege, Rind, Pferd, Affe oder Mensch und sind gegebenenfalls immortalisiert.

Die Erfindung betrifft weiterhin die Verwendung eines transgenen Säugetiers oder einer Zellinie gemäß obiger Definition als Testsystem für pharmakologische, medizinische, insbesondere diagnostische, therapeutische oder gentherapeutische Untersuchungen von Dysfunktionen der glatten Gefäßmuskeln, insbesondere Restenose.

Die gentherapeutische Anwendung des erfindungsgemäßen Genexpressionssystems erfolgt bevorzugt dadurch, daß das Konstrukt in einem pharmazeutisch akzeptablen Träger einem Säuger, insbesondere einem Menschen, über die Blutbahn in das arterielle oder venöse (Koronar-)System appliziert wird. Die Applikation der erfindungsgemäßen Nukleinsäurekonstrukte, gegebenenfalls in Kombination mit den oben beschriebenen Virusvektoren oder Liposomen, erfolgt im allgemeinen intravenös (i. v.), z.B. mit Hilfe eines Katheters. Vorteilhaft ist beispielsweise die direkte Infusion des erfindungsgemäßen Nukleinsäurekonstruktes, wie z.B. in Form rekombinanter Viren, in die Koronararterien des Patienten ("Percutaneous Coronary Gene Transfer", PCGT). Insbesondere ist die Applikation der erfindungsgemäßen Nukleinsäurekonstrukte, vor allem in Form rekombinanter Viren mit Hilfe eines Ballonkatheters, wie z.B. bei Feldman et al. (Feldman, L.J. et al. (1994) JACC 235A, 906-34) beschrieben, möglich. Liposomengebunden erfindungsgemäße Nukleinsäurekonstrukte sind nach bekannten Verfahren herstellbar (vgl. z.B. DE 44 11 402; Felgner, et al., (1987) Proc. Natl. Acad. Sci. USA, 84, 7413-7417).

Ein weiterer bevorzugter Gegenstand der Erfindung betrifft somit die Verwendung eines Nukleinsäurekonstruktes gemäß obiger Definition zur Herstellung eines pharmazeutischen Mittels zur gentherapeutischen Behandlung von Dysfunktionen der glatten Gefäßmuskeln.

Ein weiterer Gegenstand der vorliegenden Erfindung ist auch ein Arzneimittel enthaltend ein erfindungsgemäßes Nukleinsäurekonstrukt und gegebenenfalls einen pharmazeutischen Träger, der beispielsweise eine physiologische Pufferlösung, vorzugsweise mit einem pH von ungefähr 6,0 bis ungefähr 8,0, vor allem von ungefähr 6,8 bis ungefähr 7,8, insbesondere ungefähr 7,4 und/oder einer Osmolarität von ungefähr 200 bis ungefähr 400 milliosmols pro Liter (mosm/L), vorzugsweise von ungefähr 290 bis ungefähr 310 mosm/L enthält. Daneben kann der pharmazeutische Träger auch noch geeignete Stabilisatoren, wie z.B. Nukleaseinhibitoren, vorzugsweise Komplexbildner wie EDTA, und/oder andere dem Fachmann bekannte Hilfsstoffe enthalten.

Diese Erfindung bietet den überraschenden Vorteil, daß erstmals eine spezifische genetische Modifikation der glatten Gefäßmuskulatur adulter Lebewesen in vivo ermöglicht wird, wobei ein beliebiges Gen, gekoppelt an die regulatorische DNA-Sequenz von SMHC, spezifisch exprimiert werden kann.

Die Erfindung wird in den nachfolgenden Ausführungsbeispielen näher erläutert. Dabei wird Bezug genommen auf die beiliegenden Figuren. Dabei zeigt:
- Figur 1: von 5' in 3' Richtung die DNA-Squenz des 2.301 kb SMHC-Promotors (entsprechend dem Fragment -2305 bp bis -4bp).
- Figur 2: die schematische Herstellung des 5kb Mikroinjektionskonstruktes 2.3SMHC-LUC. (A) zeigt den Aufbau des Plasmids pGL2-Basic (Promega) welches die kodierende Sequenz des Markers Luciferase enthält. (B) zeigt die genomische Struktur der 5'-Region des Kaninchen SMHC-Gens einschließlich der Exons 1 und 2. Die 5'-regulatorische 2,301 kb Region enthält die TATA Box, zwei CCTCCCC Sequencen, erforderlich zur SMHC Expression, drei CArG-ähnliche Elemente, einen Enhancer (Vascular Smooth Muscle Enhancer, VSME) und ein negatives regulatorisches Element (NRE, mit * gekennzeichnet), welches mit dem dem Enhancer überlappt und einen Repressor der Transkription in nicht-glatten Muskelzellen darstellt. Das Konstrukt pGL2.3SMHC-Luc (C) wird hergestellt indem man die 2,301 kb lange regulatorische DNA Sequenz des SMHC-Promotors mit SalI und BssHII ausschneidet und in die HindIII-Schnittstelle des Vektors pGL2 Basic subkloniert. Zur Mikroinjektion wird das 5,0 kb lange 2.3SMC-Luciferase (2.3SMHC-LUC) Fragment mit SalI herausgeschnitten. Als Sonde für die Southern Blot-Hybridisierung wird das 1.7 kb Smal-EcoNI Fragment aus dem pGL2-Basic Vektor eingesetzt.
Schwarze Quadrate bezeichnen die Exons 1 und 2. Das graue Quadrat (-1,225 kb bis -1,332 kb) steht für die Enhancer-Region; und die gestreiften Quadrate bei -89 und -63 bp symbolisieren die für die SMHC-Expression erforderlichen CCTCCC-Sequenzen.
SV40 = Simian Virus 40, kb = Kilobasen; SMHC = smooth muscle myosin heavy chain; PolyA = Polyadenylierungssignal; Restriktionsenzyme: HindIII, SmaI, EcoRI, EcoNI, Sal I.

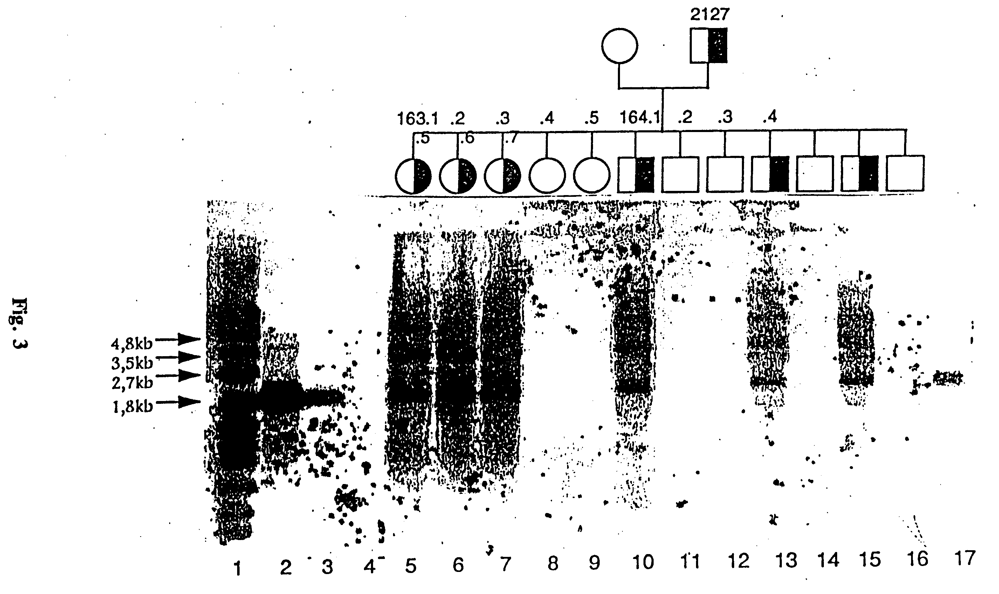

- Figur 3: die Identifikation transgener F1-Tiere mittels Southern Blot von je 10 µg EcoRI geschnittener genomischer DNA aus Mausschwanzbiopsien von Nachkommen des Founders 2127. Die Hybridisierung erfolgte mit einer Digoxigenin markierten 1,7 kb langen Luciferase-Sonde (s. Fig. 2). Positive Tiere sind an drei Banden zu erkennen. Die untere Bande besteht aus dem 1,8 kb großen SMHC-Promotor-Luciferase-Fragment (vgl. Fig. 2), die obere und mittlere kommen durch die Integration mehrerer Mikroinjektionskonstrukte zustande. Spur 1 enthält den links im Bild erläuterten Längenmarker. Spur 2 bis 4 enthält Luciferasefragment in absteigenden Konzentrationen von 100pg, 10 pg und 1 pg. Die Spuren 5 bis 17 enthalten die genomische DNA der F1-Tiere.

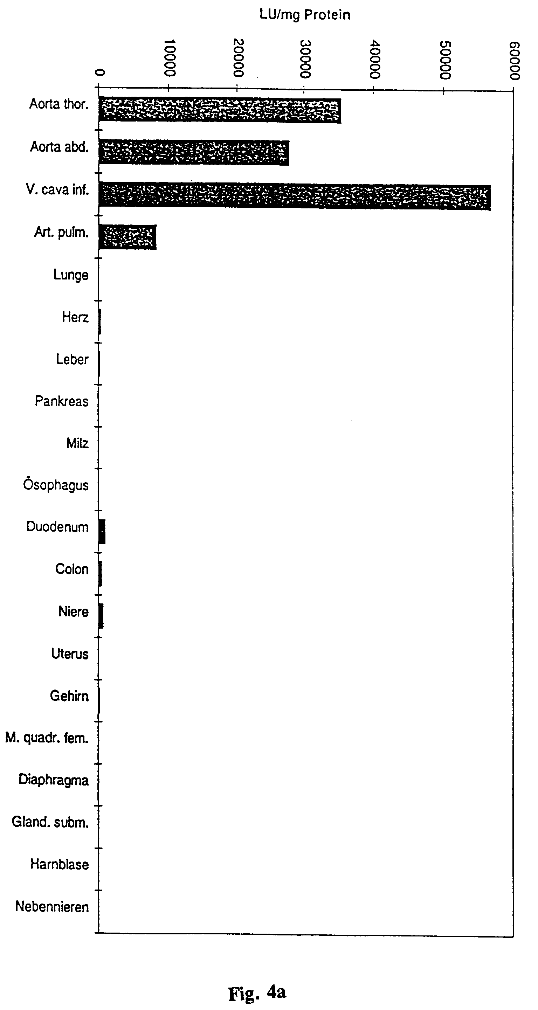

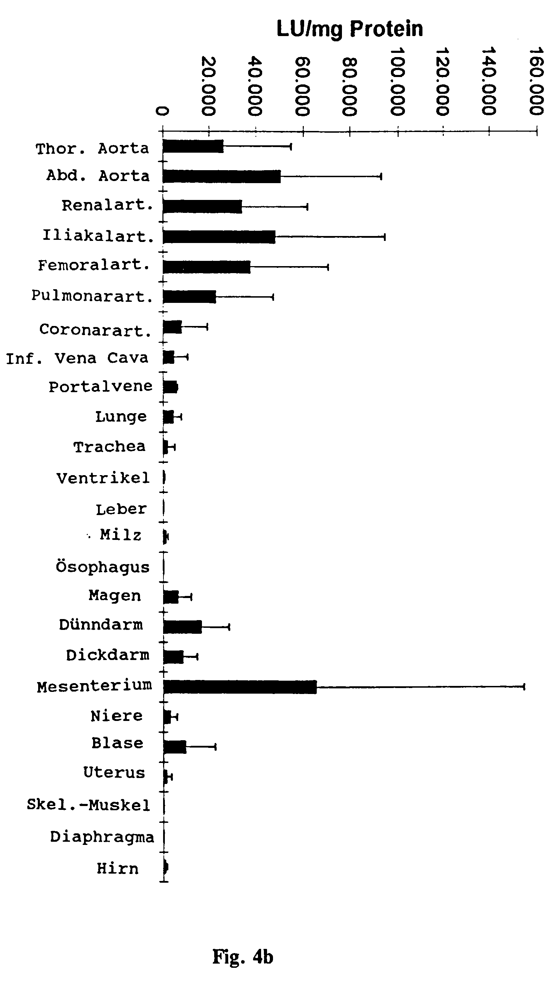

- Figur 4: a) die gefäß-spezifische Luciferase-Expression in verschiedenen Organen adulter Mäusen. Angegeben ist die spezifische Lichtaktivität (LU) in repräsentativen Organen für die transgene Mauslinie 2127, bezogen auf den Gesamtproteingehalt der Probe in Milligramm
b) Ergebnisse eines vergleichbaren Experiments. Es sind die Mittelwerte und Stardardabweichnungen für Lichteinheiten [LU] pro mg Protein angegeben. Die Gewebeproben wurden aus 10 heterozygoten adulten Tieren der Mauslinie 2127 erhalten.

Werden in den folgenden Ausführungsbeispielen keine anderslautenden Angaben gemacht, können diese unter Befolgung aus dem Stand der Technik bekannter Verfahrensmaßnahmen vom Durchschnittsfachmann ohne unzumutbaren Aufwand durchgeführt werden. Bezüglich Verfahrensmaßnahmen zur Herstellung der erfindungsgemäß verwendeten Nukleinsäurekonstrukte sei insbesondere verwießen auf Maniatis, T., et al., Molecular Cloning (1982); Cold Spring Harbor Laboratory.

### Beispiel 1:

### Klonierung des SMHC-Promotors

Die Klonierung des SMHC-Promotors erfolgte wie von Solway, J. et al. beschrieben in J. Biol. Chem. 1995; 270: 13460-13469. Ein 2,301 kb Promotorfragment (-2,305 kb bis -4 bp stromaufwärts vom Transkriptionsstart gelegen; vgl. Fig.1) wurde, wie von Kallmeier, R.C. et al. in J. Biol. Chem. 1995; 270: 30949-30957 beschrieben, mit SalI und BssHII isoliert. Das Promotorfragement wurde mit HindIII-Linkern versehen und in die HindIII-Schnittstelle des Luciferase-Reportervektors pGL2 Basic (Promega) subkloniert und somit an das Luciferase-Gen und das SV40-Polyadenylierungssignal gekoppelt. Auf diese Weise erhält man das Plasmid pGL2.3SMHC-Luc (vgl. Fig. 2).

### Beispiel 2:

Herstellung eines Mikroinjektionskonstruktes zur Erzeugung transgener Mäuse.

Das gemäß Beispiel 1 hergestellte Plasmid pGL2.3SMHC-Luc wurde mit SalI geschnitten. Auf diese Weise erhält man das ca. 5 kb groBe SMHC-Luciferase Fusionsgen 2.3SMHC-Luc (vgl. Fig. 2). Nach elektrophoretischer Reinigung des 2.3SMHC-Luc-Fragmentes erfolgt die Erzeugung transgener Mäuse, wie beispielsweise von Brem in Arzneimittelforschnung 1990, 40: 335-43 beschrieben. Ein Picoliter Injektionslösung, enthaltend 500 - 1000 Kopien des Transgens in 10 mM Tris-HCl (pH 7,5) und 0,2 mM EDTA, wird in den männlichen Pronucleus fertilisierter Mausoozyten [(HimOF1)F1 x NMRI] mikroinjiziert.

Zur Identifizierung transgener Founder und ihrer Nachkömmlinge wurde DNA aus Mausschwanzbiopsien mit Hilfe des Qiamp Tissue Kit (Qiagen, Hilden, Deutschland) extrahiert und nach PCR-Amplifikation durch nicht-radioaktive Southern-Hybridisierung unter Verwendung eines 1,8 kb SmaI-EcoNI Luciferasefragmentes, markiert mit Digoxigenin-11-dUTP (Boehringer, Mannheim, Indianapolis), wie von Schnorf et al, in Transgen Res. 1991; 1:23 bis 30 beschrieben, analysiert. Positive Tiere konnten über ein typisches Restriktionsmuster ihrer Banden nach Verdauung mit EcoRI identifiziert werden.

Positive männliche Tiere wurden für weitere Untersuchung der Onthogenie mit weiblichen Wildtyp-Partnern verkreuzt. Die Nachkommen zeigten einen Mendelschen Erbgang des Transgens, wodurch die erfolgreiche Keimbahnintegration bestätigt wurde (Fig. 3).

### Beispiel 3:

Nachweis der Reportergen-Expression in verschiedenen Geweben.

Zum Nachweis der Luciferase-Expression wurde ein repräsentativer Querschnitt verschiedener Organe adulter Mäuse analysiert.

Gewebeproben wurden dekapitierten Mäusen entnommen und sofort in flüssigem Stickstoff eingefroren. Bei der Entnahme wurde darauf geachtet, daß große vaskuläre Strukturen von Organen, die parenchymale und viscerale glatte Muskulatur enthalten, entfernt wurde. Das Gewebe wurde in flüssigem Stickstoff pulverisiert und in einer 10-fachen Menge Lysepuffer (25 mM Tricin, pH 7,8, 4 mM EGTA, 10% Glycerin, 1 mM DTT) 10 Minuten bei Zimmertemperatur homogenisiert. Nichtextrahiertes Material wurde 10 Minuten bei 12000 g abzentrifugiert. Der Überstand wurde in ein frisches Probenröhrchen überführt.

Die Luciferase-Aktivität wurde unter Verwendung des Luciferase-Testsystems von Boehringer Mannheim, Indianapolis, bestimmt. 100 µl Luciferinlösung wurden mit 50 µl Proteinextrakt versetzt. Die Lichtemission wurde über einen Zeitraum von 20 Sekunden mit einem Lumat LD 9501 Luminometer (Berthold, Bad Wildbad, Deutschland) bestimmt. Jeweils drei Messungen wurden für eine Probe durchgeführt. Die Enzymaktivitäten wurden bezogen auf den Gesamtproteingehalt des Zellextraktes in mg, bestimmt mit Hilfe der Bicinchoninsäure-Methode (Pierce, Rockford, IL., USA).

Die Ergebnisse für zwei Meßreihen sind in den Figuren 4a und 4b dargestellt.

### Versuchsergebnisse:

1. Erzeugung einer transgenen Mauszellinie, enthaltend das 2.3SMHC-Luc Transgen
   Durch Southern-Hybridisierung und PCR-Analyse wurde ermittelt, daß von 78 Nachkommen insgesamt 7 Mäuse das Transgen in ihrem Genom enthielten. Sechs dieser sieben Founder-Mäuse übertrugen 2.3SMC-Luc auf die F1-Generation nach dem Mendelschen Gesetz. Drei Zellinien (2127, 178.5, #7) exprimierten das Transgen im vaskulären System, während die drei anderen Linien nur Background-Aktivitäten zeigten. Die experimierenden Maus-Linien wurden in den homocygoten Zustand überführt und weiter charakterisiert. Die höchsten Luciferase-Aktivitäten wurden in der Mauslinie 2127 nachgewiesen.
2. Gefäß-spezifische Aktivität des 2.3 kb SMHC-Promotors in adulten Mäusen
   Es ist bekannt, daß die Aktivität des SMHC-Promotors nach der Geburt abreguliert wird. Für eine detaillierte Analyse des vom 2.3 SMHC-Promotor bedingten Expressionsmuster wurden die Lichtaktivitäten in Gewebelysaten von repräsentativen Organen aus adulten transgenen 2.3 SMHC-Luc Mäusen bestimmt (Figuren 4 a und b). Hohe Luciferase-Aktivitäten wurden in Gefäßen des arteriellen Systems, einschließlich der Koronararterien gemessen. Signifikant erhöhte Expression wurde auch im venösen System (Vena cava, Pfortader) gefunden. Keine oder lediglich Background-Aktivitäten wurden anderen Proben , wie z.B. im Herzen, Skelettmuskulatur, Leber, Ösophagus, Lunge, Trachea, Pankreas, Duodenum, Colon, Milz, Blase, Uterus, Harnblase Nebennieren und Gehirn bestimmt.
   Etwas erhöhte Background-Aktivitäten in solchen Proben können durch eine unvollständige Abtrennung der vaskulären Strukturen vor der Analyse erklärt werden. Eine Abtrennung des Mesenteriums, das den größten Teil der intestinalen Gefäßstruktur enthält, ergab ebenfalls überraschenderweise eine mit Aorta oder anderen Arterien vergleichbare Luciferase-Aktivität. Zusammen mit dem Befund, daß keine Aktivität im Ösophagus, das kein Mesenterium enthält, gemessen werden konnte, ist daraus zu schließen, daß die in einigen Versuchen beobachtete geringe intestinale Expression aus glattem Gefäßmuskel der Mesenterialgefäße stammt.
   In weiteren Versuchen (Ergebnisse nicht gezeigt) wurde die Luciferase-Genexpression in Koranararterien untersucht. Bei diesen Experimenten wurde die Koronararterie von Myokardgewebe abgetrennt. Lichtaktivität wurde ausschließlich in Proteinextrakten der Koronargefäße, nicht jedoch in Myocard-Proben nachgewiesen. Diese Ergebnisse bestätigen, daß die erfindungsgemäß verwendeten Promotorsequenzen die erforderlichen cis-Elemente enthalten, um eine Genexpression im venösen und arteriellen Gefäßsystem, einschließlich der Koronararterien, zu steuern.

Zusammenfassend ist festzustellen, daß der erfindungsgemäß bevorzugt verwendete 2.301 kb SMHC Promotor aus Kaninchen überraschenderweise alle wesentlichen regulativen Elemente enthält, die für eine spezifische Genexpression im adulten vaskulären Gefäßsystem von Arterien und Venen notwendig sind und sich somit für gentherapeutische Ansätze eignet.

## Patentansprüche

1. Nukleinsäurekonstrukt mit Spezifität für glatte Gefäßmuskelzellen, dadurch gekennzeichnet, daß es unter der Kontrolle einer regulativen Nukleotidsequenz für die Myosin-Schwere Kette der glatten Muskulatur (SMHC) eine funktionale Nukleotidsequenz umfaßt.

2. Nukleinsäurekonstrukt nach Anspruch 1, dadurch gekennzeichnet, daß die regulative Nukleotidsequenz eine SMHC-Promotorsequenz umfaßt.

3. Nukleinsäurekonstrukt nach Anspruch 2, dadurch gekennzeichnet, daß der SMHC Promotor die in Figur 1 angegebene Nukleotidsequenz von Nukleotidrest +1 bis Nukleotidrest +2301 oder ein funktionales Äquivalent davon umfaßt.

4. Nukleinsäurekonstrukt nach einem der Ansprüche 1 bis 3, dadurch gekennzeichnet, daß die funktionale Nukleotidsequenz
a) für wenigstens ein in den Gefäßmuskelzellen zu exprimierendes heterologes oder homologes Genprodukt kodiert; oder
b) eine therapeutisch wirksame Nukleotidsequenz darstellt.

5. Nukleinsäurekonstrukt nach Anspruch 4, dadurch gekennzeichnet, daß die kodierende Nucleotidsequenz für ein Markergen oder ein therapeutisch wirksames Genprodukt, insbesondere für ein antiproliferativ wirkendes Genprodukt kodiert.

6. Nukleinsäurekonstrukt nach Anspruch 5, dadurch gekennzeichnet, daß das antiproliferativ wirkende Genprodukt ausgewählt ist unter Toxinen und Zellzyklus-Inhibitoren.

7. Nukleinsäurekonstrukt nach einem der vorhergehenden Ansprüche, dadurch gekennzeichnet, daß es weitere regulative Nucleotidsequenzen umfaßt, ausgewählt unter Leadersequenzen, Enhancersequenzen, Polyadenylierungssignalen und die Expressionsrate steuernde Sequenzen.

8. Nukleinsäurekonstrukt nach einem der vorhergehenden Ansprüche, dadurch gekennzeichnet, daß es in 3'-Position zur kodierenden Sequenz ein SV40-Polyadenylierungssignal umfaßt.

9. Vektorsystem, umfassend eine Nukleinsäurekonstrukt nach einem der vorherigen Ansprüche.

10. Vektorsystem nach Anspruch 9, nämlich ausgewählt unter viralen und nicht-viralen Vektoren, gegebenenfalls in Kombination mit Liposomen.

11. Transgenes Säugetier, dadurch gekennzeichnet, daß es transformiert ist mit einer Nukleotidsequenz, umfassend wenigstens ein Nukleinsäurekonstrukt nach Anspruch 1 bis 8 oder wenigstens einen Vektor nach Anspruch 9 und 10.

12. Transgenes Säugetier nach Anspruch 11, ausgewählt unter Maus, Ratte, Hamster, Kaninchen, Katze, Hund, Schwein, Schaf, Ziege, Rind, Pferd und Affe.

13. Verfahren zur Herstellung eines transgenen Säugetiers nach einem der Ansprüche 11 und 12, dadurch gekennzeichnet, daß man
eine befruchtete Eizelle des Säugetiers gewinnt;
in den männlichen Pronucleus eine Lösung mikroinjiziert,
welche wenigsten ein Nukleinsäurekonstrukt nach einem der Ansprüche 1 bis 8 oder wenigstens einen Vektor nach Anspruch 9 oder 10 umfaßt;
die transfizierte Eizelle in das Ovidukt eines scheinträchtigen Wirts implantiert; und
das transgene Tier durch den Wirt austragen läßt.

14. Zellinie der glatten Gefäßmuskulatur, dadurch gekennzeichnet, daß die Zellinie transformiert ist mit einer Nukleotidsequenz, umfassend wenigstens ein Nukleinsäurekonstrukt nach Anspruch 1 bis 8 oder wenigstens einen Vektor nach Anspruch 9 oder 10.

15. Zellinie anch Anspruch 14, dadurch gekennzeichnet, daß sie abgeleitet ist aus embryonalen Stammzellen von Maus, Ratte, Hamster, Kaninchen, Katze, Hund, Schwein, Schaf, Ziege, Rind, Pferd, Affe oder Mensch.

16. Verwendung eines transgenen Säugetiers nach einem der Ansprüche 11 und 12 oder einer Zellinie nach einem der Ansprüche 14 und 15 als Testsystem für pharmakologische, medizinische, insbesondere diagnostische, therapeutische oder gentherapeutische Untersuchungen von Dysfunktionen der glatten Gefäßmuskeln.

17. Verwendung nach Anspruch 16 dadurch gekennzeichnet, daß die Dysfunktion eine Restenose umfaßt.

18. Verwendung eines Nukleinsäurekonstrukts nach Anspruch 1 bis 8 oder eines Vektors nach Anspruch 9 oder 10 zur Herstellung eines pharmazeutischen Mittels zur gentherapeutischen Behandlung von Dysfunktionen der glatten Gefäßmuskeln.

19. Verwendung nach Anspruch 18, wobei man das Konstrukt oder den Vektor, vorzugsweise mit Hilfe eines Katheters, dem arteriellen oder venösen Koronarsystem appliziert.

20. Verwendung einer Nukleinsäurekonstrukt nach Anspruch 1 bis 8 oder eines Vektors nach Anspruch 9 oder 10 zur Selektion oder Immortalisierung glatter Gefäßmuskelzellen.

21. Pharmazeutisches Mittel, enthaltend in einem pharmazeutisch akzeptablen Träger eine gentherapeutisch wirksame Menge wenigstens eines Nukleinsäurekonstruktes nach einem der Ansprüche 1 bis 8 oder wenigstens eines Vektors nach Anspruch 9 oder 10.
